# EUROPEAN PATENT SPECIFICATION

(11) **EP 3 103 614 B1**
(45) Date of publication and mention of the grant of the patent: **16.05.2018**
(21) Application number: 15746251.6
(22) Date of filing: 04.02.2015
(51) Int. Cl.: B29C 45/14, B29C 45/16, B29C 45/26, B29K 105/20, B29L 23/00, B29L 31/00, A61M 39/08

(54) **METHOD FOR PRODUCING A TUBE AND MOULDING DIE**
VERFAHREN ZUR HERSTELLUNG EINES ROHRS UND ENTSPRECHENDES FORMWERKZEUG/MATRIZE
PROCÉDÉ DE FABRICATION DU TUBE ET UN MOULE/MATRICE

(30) Priority: 07.02.2014 JP 2014022672
(43) Date of publication of application: 14.12.2016
(73) Proprietor: Tatsuta Electric Wire & Cable Co., Ltd., Higashiosaka-shi, Osaka 578-8585 (JP)
(72) Inventor: OHNISHI, Hisafumi, Higashiosaka-shi Osaka 578-8585 (JP); YOSHINO, Shinji, Kizugawa-shi Kyoto 619-0216 (JP); HIRANO, Yoshio, Kizugawa-shi Kyoto 619-0216 (JP)
(74) Representative: Bittner, Thomas L.
(86) International application number: PCT/JP2015/053082
(87) International publication number: WO 2015/119143

(56) References cited:
- JP-A- H04 174 663
- JP-A- 2005 256 934
- JP-A- 2005 256 934
- JP-A- 2008 252 666
- JP-A- 2010 048 333
- US-A- 4 174 367
- US-A- 5 245 955
- US-A1- 2003 015 816

## Description

The present invention relates to a tube, a method for producing a tube, and a mold.

As one of medical instruments, a cannula which is a tubular component is known. In general, the cannula is made of resin and is inserted into a lumen, a blood vessel, or the like of a patient for excreting a body fluid or for instillation of a drug solution, a contrast medium, or the like.

Examples of a method for molding a medical tube (such as a cannula) which is made of resin encompass molding methods such as extrusion molding and injection molding. JP 4-174663 discloses a method for producing a medical tube by injection molding.

In a case where a medical tube is molded by injection molding, it is possible to collectively form, in an injection molding step, another member to be attached to the medical tube. This brings an advantage of simplifying production processes. Furthermore, the injection molding has an advantage that a tube which has a complicated shape and is difficult to produce by extrusion molding can be produced with high accuracy.

A medical tube which is made of only resin is bent in a folded manner by applied force, and a hollow section at the bent part becomes narrower. This disturbs transfer of blood, a drug solution, or the like and may cause a problem that such a liquid cannot be transferred at all depending on circumstances.

In order to solve the problem, in the medical tube of JP 4-174663, a reinforcing body having a spiral shape is embedded in a thick wall part (wall section). This makes it possible to cause the medical tube to be hardly bent in a folded manner and consequently to restrain the hollow section from being narrowed, and further to prevent the disturbance in transfer of a drug or the like.

A method of JP 4-174663 for producing a medical tube is a production method in which (i) firstly a core pin is covered with a silicone rubber tube which has been molded in advance, (ii) secondly the silicone rubber tube is covered with a reinforcing body which is made of stainless steel and has a spiral shape, and (iii) thirdly the core pin covered with the reinforcing body and the silicone rubber tube is set in a cavity of a mold and injection molding is carried out. By the production method, a tube can be produced in which the reinforcing body having the spiral shape is embedded in a wall section.

Moreover, JP 2013-180545 discloses a production method in which (i) a primary molded body corresponding to a lower-half part of a tube is molded with use of a mold for first molding and a mold for closing and (ii) a secondary molded body corresponding to an upper-half part of the tube is molded with use of a mold for second molding instead of the mold for closing. With the production method, a tube having a thin wall can be molded with high accuracy.

Document JP 2005 25 69 34 refers to an electric fusion joint being formed by providing gasoline resistance and its manufacturing method, capable of connecting a polyethylene multilayer tube having a barrier layer of an ethylene-vinyl a copolymer in the middle so as to obtain gasoline resistance.

Document US 4,174,367 refers to a form tool for the production of a hose piece from an injectable material with wire-, rope- or cable-, or thread- shaped reinforcement inserts made of metal, textile or the like, the reinforcement inserts extending in the circumferential direction of the hose piece, comprising a mould sleeve and a mould core, which form an annular cylindrical hollow mould space therebetween, whereby on the periphery of the mould core, extending in the axial direction of the latter, supporting ribs for distancing of the reinforcement inserts are arranged spaced from the periphery of the mould core, and whereby the supporting ribs have recesses in which there engage the reinforcement inserts which are wound on the supporting ribs. The form tool is formed as an injection mould with an injection position arranged on a face edge of the hollow mould space, wherein the recesses are respectively formed sawtooth-like with a steep flank supporting the reinforcement inserts in the axial injection direction and with a flank ascending flatly inclined counter to the direction of injection.

Document US 2003/0015816 A1 refers to methods and devices from manufacturing a conduit for placing a target vessel in fluid communication with a source of blood, such as a heart chamber containing blood. The conduit includes first and second portion adapted to be placed in fluid communication with a heart chamber and a target vessel. The conduit lies on the exterior of the myocardium between the blood source and the target vessel and delivers blood in multiple directions within the lumen of the target vessel. The conduit, which may be formed of any suitable synthetic vascular graft material, is generally T-shaped with the leg having two free ends disposed in the target vessel, preferably being secured thereto via a suture-free attachment. The conduit comprises vascular graft material and may be manufactured various ways, such as molding a conduit from any suitable biocompatible material or fabricating a conduit from one or more pieces of vascular graft material.

### Summary of Invention

However, in the production method of JP 4-174663, the silicone rubber tube is prepared in advance, the silicone rubber tube is covered with the reinforcing body having a spiral shape, and further the injection molding is carried out. As such, the processes are complicated as compared with processes for producing a tube having no reinforcing body.

Moreover, in the production method of JP 4-174663, the silicone rubber tube is covered with the reinforcing body, and therefore the reinforcing body is designed to have an inner diameter which is larger than an outer diameter of the silicone rubber tube. This causes a problem that it is difficult to control arrangement of the reinforcing body so that a central axis of the silicone rubber tube conforms to a central axis of the reinforcing body. From this, the reinforcing body may deviate in position in the wall section of the obtained medical tube in a radial direction of the medical tube (i.e., in a direction perpendicular to a longitudinal direction of the medical tube). Further, the reinforcing body may be partially exposed in a surface of the tube, depending on circumstances.

In order to surely embed the reinforcing body in the wall section so that the reinforcing body is not exposed, it is necessary to design the wall section to be thick in advance so as to prevent the reinforcing body from being exposed even in a case where thickness deviation occurs in the wall section when the injection molding is carried out. As a result, an outer diameter of the medical tube becomes larger than necessary with respect to an inner diameter, and this increases a burden to a patient.

Further, in a case where a gap between the silicone rubber tube and the reinforcing body is large in a state in which the silicone rubber tube is covered with the reinforcing body, the reinforcing body is moved from a predetermined position by pressure applied to the reinforcing body in the injection molding. In particular, the reinforcing body is moved in a longitudinal direction of the silicone rubber tube by injection pressure and accordingly deviates from the predetermined position.

In view of this, in order to reduce a gap between the silicone rubber tube and the reinforcing body, it may be considered that the reinforcing body is designed to have an inner diameter which is less different from an outer diameter of the silicone rubber tube. However, in a case where the reinforcing body and the silicone rubber tube are designed as above described, it becomes difficult to put the reinforcing body over a surface of the silicone rubber tube at a predetermined position, and this leads to a problem of taking time to carry out this step.

Moreover, the production method of JP 2013-180545 is a complicated production method which (i) includes the step of molding the lower-half part of the tube and the step of molding the upper-half part of the tube and, further, (ii) requires three kinds of molds. In a case where a tube in which a reinforcing body is embedded in a wall section is produced by using the production method of JP 2013-180545, processes become further complicated, and the number of required molds further increases.

The present invention is accomplished in view of the problems, and its object is to provide (i) a tube which has a small outer diameter and is hardly bent in a folded manner, (ii) a tube production method for molding the tube with simple processes, and (iii) a mold for use in molding of the tube.

### Solution to Problem

In order to attain the object, a tube production method in accordance with an aspect of the present invention is a method for producing a tube which includes a wall section, a hollow section, and a reinforcing member, the reinforcing member being provided in the wall section and having a ring shape, a wide-ring shape, or a spiral shape, the method including the steps of: (a) forming a primary molded body, in which the reinforcing member is provided along an outer peripheral surface of a tubular resin, by carrying out resin molding while the reinforcing member and a core pin for forming the hollow section are placed in a cavity of a first mold; and (b) covering the reinforcing member by carrying out resin molding while the primary molded body is placed in a cavity of a second mold which has an inner diameter larger than an inner diameter of the first mold.

In order to attain the object, a mold in accordance with an aspect of the present invention is a mold for forming a tube which includes a wall section, a hollow section, and a reinforcing member, the reinforcing member being provided in the wall section and having a ring shape, a wide-ring shape, or a spiral shape, said mold including: a first mold; and a second mold, an inner diameter of the second mold being larger than an inner diameter of the first mold, and the first mold having an inner wall surface which is provided with a plurality of hole-like recessed parts.

In order to attain the object, a tube in accordance with an aspect of the present invention is tube including: a wall section; a hollow section; a reinforcing member which is provided in the wall section and serves as at least one ring-shaped member, at least one wide-ring-shaped member, or at least one turn of a spiral-shaped member; a first resin layer; and a second resin layer which is provided on an outer side of the first resin layer, in a cross section of the tube taken along a plane including an axis direction of the tube, a cross section of the reinforcing member being located in a boundary between the first resin layer and the second resin layer, a part of the first resin layer, which part is located at least at a certain part of the tube, being thicker than other parts of the first resin layer, and a part of the second resin layer, which part is located at least at a certain part of the tube, being thinner than other parts of the second resin layer.

Note that, in the present invention, the term "mold" means a "mold" used in resin molding, and a material of the mold can be selected as appropriate from metal materials and nonmetal materials in accordance with a resin molding method.

### Advantageous Effects of Invention

According to an aspect of the present invention, it is possible to provide (i) a tube which has a thin wall section and is hardly bent in a folded manner, (ii) a tube production method for molding the tube with simple processes, and (iii) a mold for use in molding of the tube.

### Brief Description of Drawings

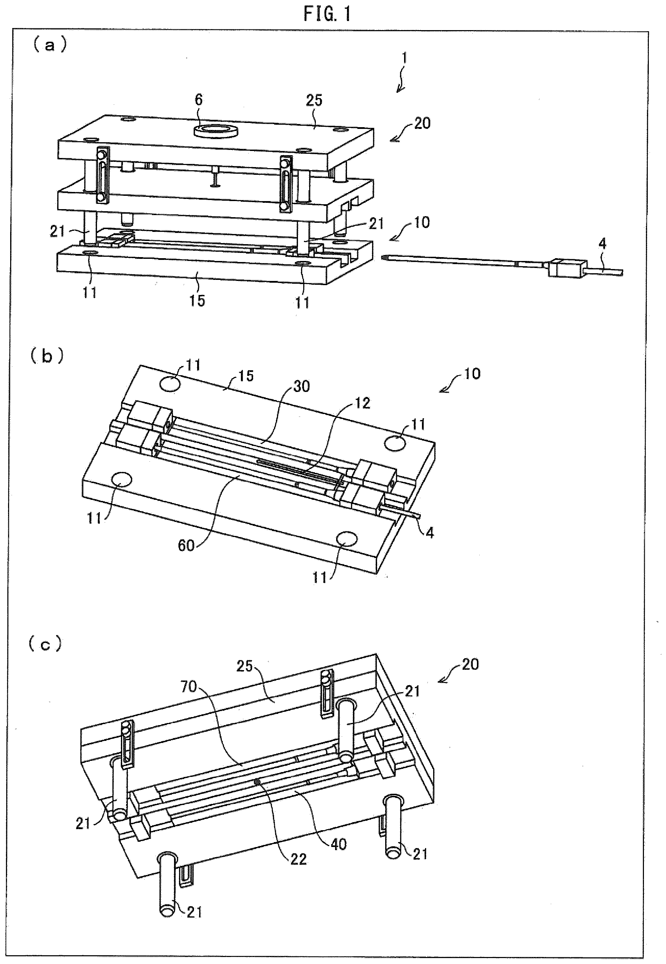

Fig. 1 is a perspective view partially illustrating a resin molding mold in accordance with Embodiment 1 of the present invention. (a) of Fig. 1 illustrates a state where a resin receiving side mold and an opposite side mold face each other, (b) of Fig. 1 illustrates the opposite side mold, and (c) of Fig. 1 illustrates the resin receiving side mold.

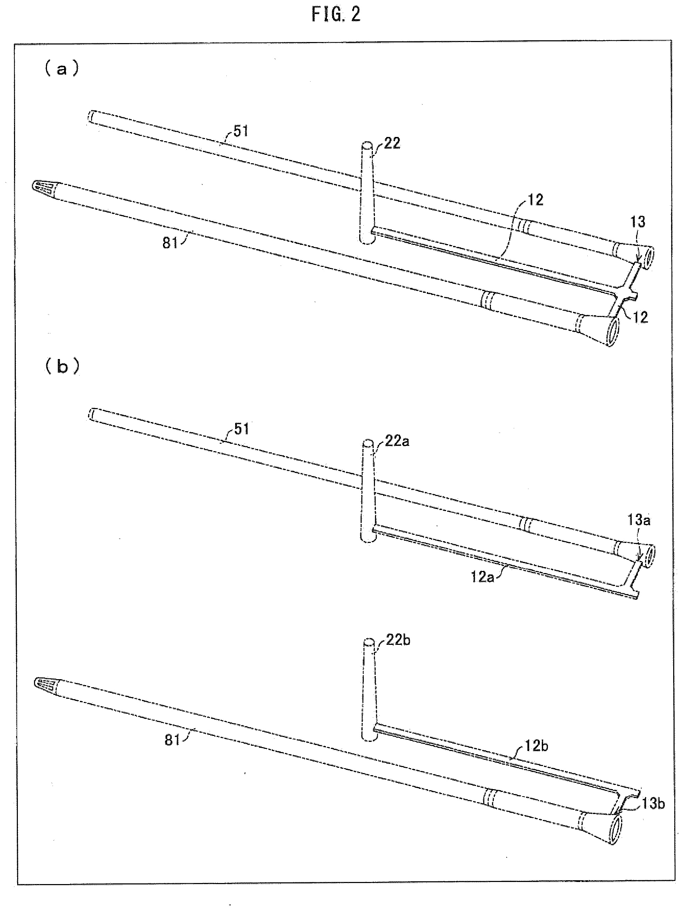

Fig. 2 is a virtual perspective view illustrating a path through which molten resin flows. (a) of Fig. 2 is a virtual perspective view illustrating a path through which molten resin flows in the mold in accordance with Embodiment 1, and (b) of Fig. 2 is a virtual perspective view illustrating a path through which molten resin flows in a mold in accordance with a modification example.

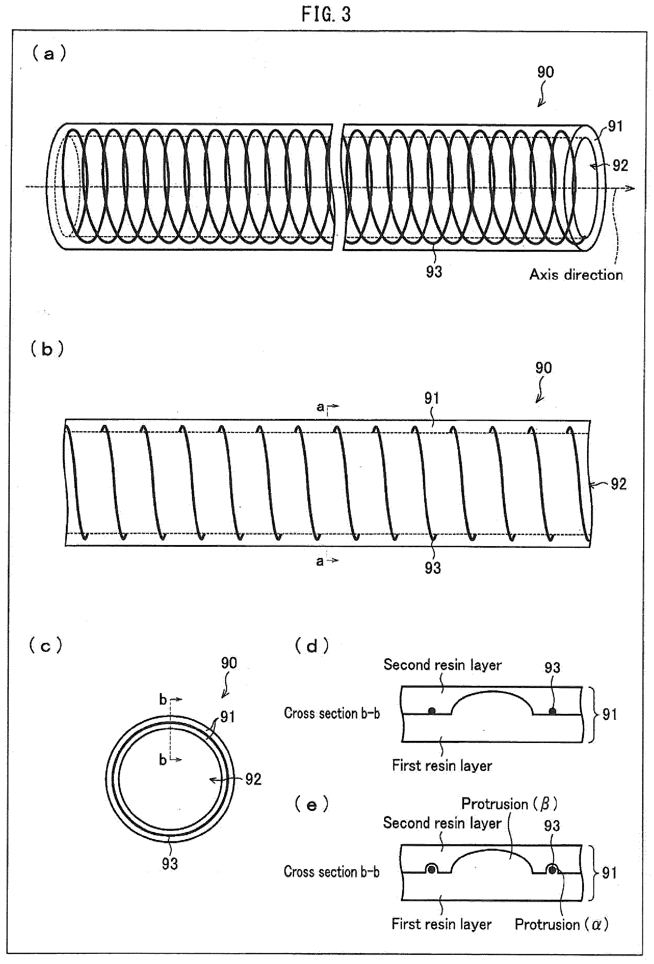

Fig. 3 is a view schematically illustrating a tube in accordance with Embodiment 1 of the present invention. (a) of Fig. 3 is a perspective view, (b) of Fig. 3 is a partial lateral view, (c) of Fig. 3 is a cross-sectional view taken along the line a-a in (b) of Fig. 3, and each of (d) and (e) of Fig. 3 is a partial cross-sectional view taken along the line b-b in (c) of Fig. 3.

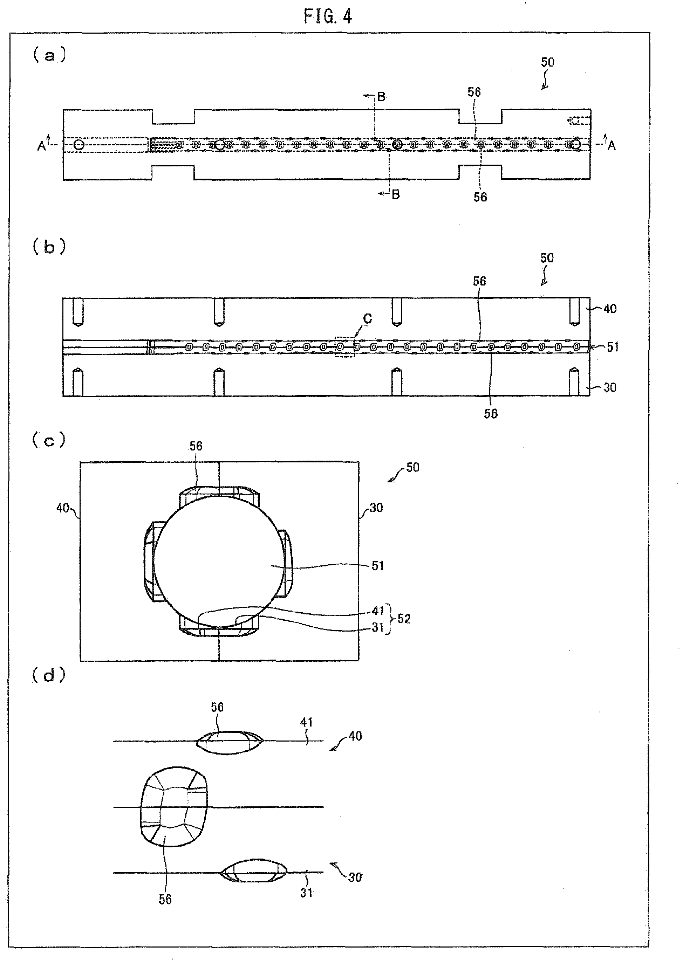

Fig. 4 is a view schematically illustrating a first mold in accordance with Embodiment 1 of the present invention. (a) of Fig. 4 is a plan view, (b) of Fig. 4 is a cross-sectional view taken along the line A-A in (a) of Fig. 4, (c) of Fig. 4 is a cross-sectional view taken along the line B-B in (a) of Fig. 4, and (d) of Fig. 4 is an enlarged view illustrating a part C in (b) of Fig. 4.

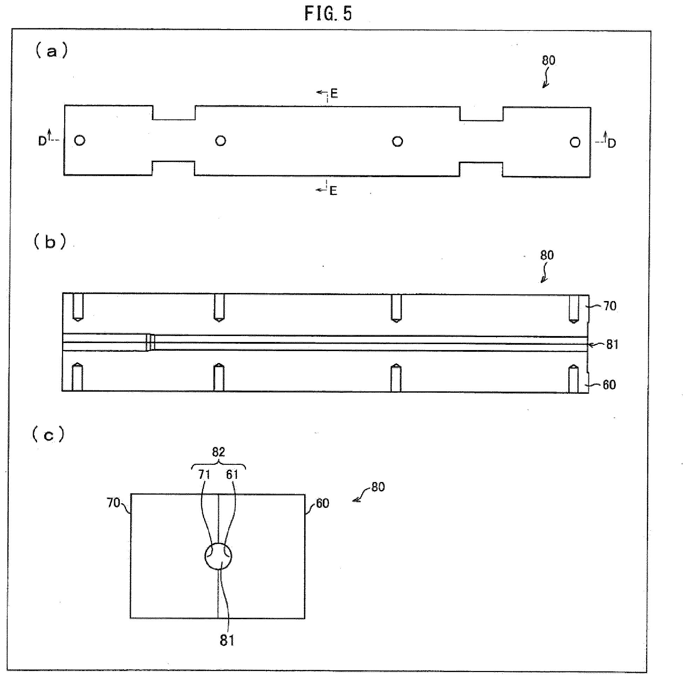

Fig. 5 is a view schematically illustrating a second mold in accordance with Embodiment 1 of the present invention. (a) of Fig. 5 is a plan view, (b) of Fig. 5 is a cross-sectional view taken along the line D-D in (a) of Fig. 5, and (c) of Fig. 5 is a cross-sectional view taken along the line E-E in (a) of Fig. 5.

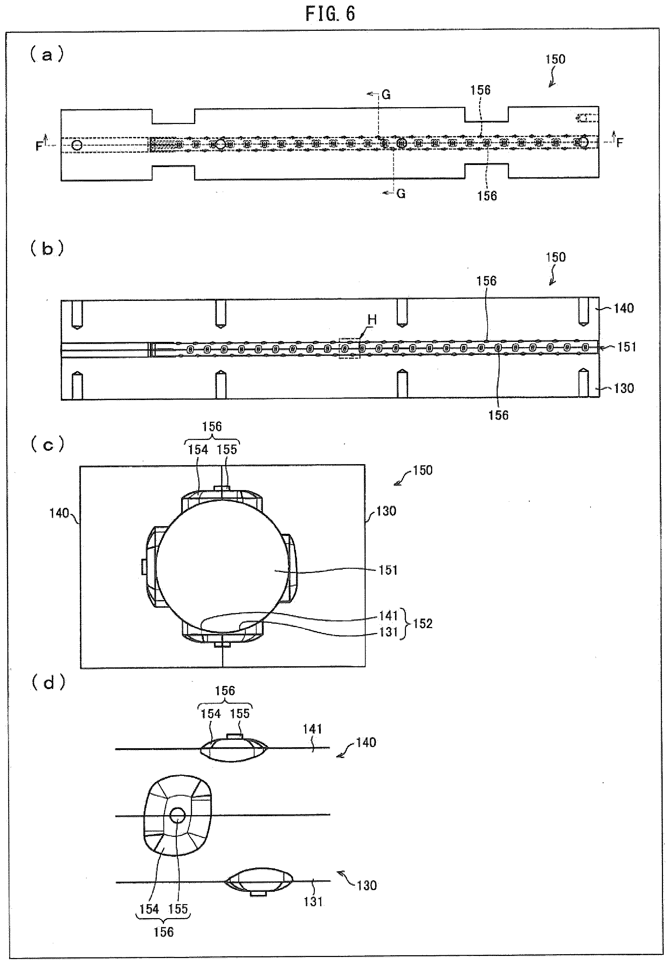

Fig. 6 is a view schematically illustrating a first mold in accordance with Embodiment 2 of the present invention. (a) of Fig. 6 is a plan view, (b) of Fig. 6 is a cross-sectional view taken along the line F-F in (a) of Fig. 6, (c) of Fig. 6 is a cross-sectional view taken along the line G-G in (a) of Fig. 6, and (d) of Fig. 6 is an enlarged view illustrating a part H in (b) of Fig. 6.

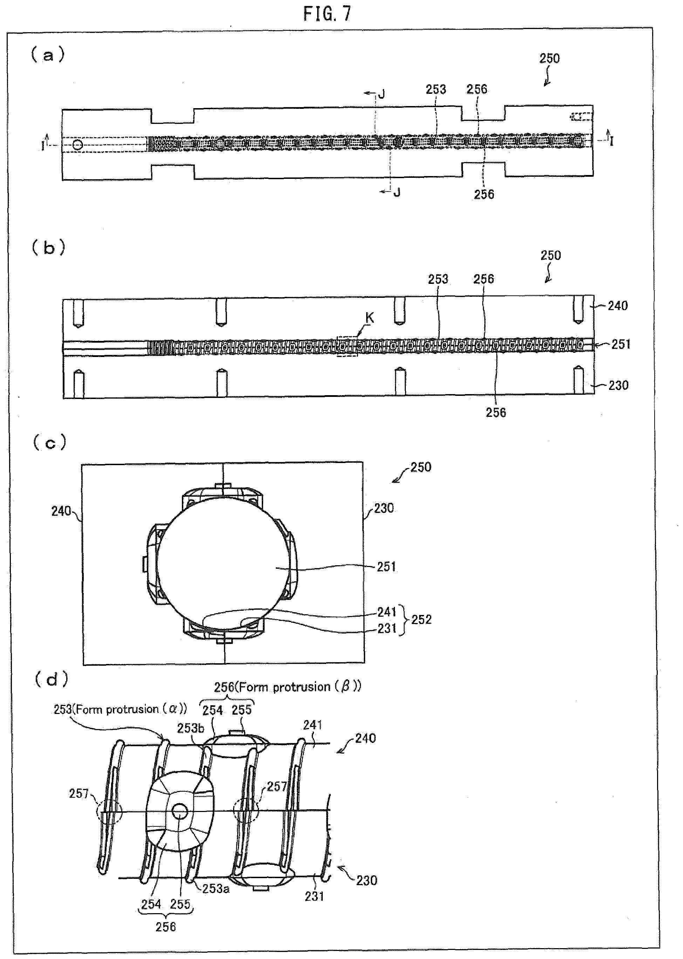

Fig. 7 is a view schematically illustrating a first mold in accordance with Embodiment 3 of the present invention. (a) of Fig. 7 is a plan view, (b) of Fig. 7 is a cross-sectional view taken along the line I-I in (a) of Fig. 7, (c) of Fig. 7 is a cross-sectional view taken along the line J-J in (a) of Fig. 7, and (d) of Fig. 7 is an enlarged view illustrating a part K in (b) of Fig. 7.

### Description of Embodiments

### [Embodiment 1]

The following description will discuss, with reference to Figs. 1 through 5, details of a method for producing a tube, a tube, and a mold in accordance with an embodiment of the present invention.

### <Resin molding mold>

Fig. 1 is a perspective view partially illustrating a resin molding mold 1 in accordance with Embodiment 1. (a) of Fig. 1 illustrates a state where a resin receiving side mold and an opposite side mold face each other, (b) of Fig. 1 illustrates the opposite side mold, and (c) of Fig. 1 illustrates the resin receiving side mold.

Examples of resin molding which can be employed in the present invention encompass injection molding, vacuum injection, and the like. In the following descriptions, an embodiment of injection molding is first described, and an embodiment of vacuum injection will be described later.

An injection molding machine includes a resin molding mold 1 and an injection unit (not illustrated). The injection unit has a configuration which is identical with that of a conventional injection unit, and is therefore not illustrated in a drawing and not described in detail.

As illustrated in (a) of Fig. 1, the resin molding mold 1 includes an opposite side mold 10 and a resin receiving side mold 20 which are arranged so as to face each other. Moreover, as illustrated in (b) of Fig. 1, the opposite side mold 10 includes an opposite side mold plate 15, and a lower mold 30 for primary molding and a lower mold 60 for secondary molding which are provide on the opposite side mold plate 15. Further, as illustrated in (c) of Fig. 1, the resin receiving side mold 20 includes a resin receiving mounting plate 25 (base plate), and an upper mold 40 for primary molding and an upper mold 70 for secondary molding which are provided on the resin receiving mounting plate 25.

As illustrated in (a) and (c) of Fig. 1, the resin receiving mounting plate 25 is provided with (i) a locate ring 6 for making alignment with the injection unit easy and (ii) four guide pins 21 which are provided at respective four corners of a surface facing the opposite side mold 10. Moreover, as illustrated in (b) of Fig. 1, the opposite side mold plate 15 has guide pin bushes 11 into which the guide pins 21 are to be respectively inserted. By inserting the guide pins 21 into the respective guide pin bushes 11, the resin receiving side mold 20 and the opposite side mold 10 are closely brought into contact while being aligned with each other. As such, clamping of the resin molding mold 1 is carried out.

As illustrated in (b) of Fig. 1, the opposite side mold 10 has a runner 12 which is provided on a surface facing the resin receiving side mold 20. Moreover, the resin receiving side mold 20 has a sprue 22 which is to communicate with the runner 12 when the resin molding mold 1 is clamped. The sprue 22 passes through the resin receiving side mold 20, and molten resin is introduced from a nozzle of the injection unit via a sprue bush.

When the resin molding mold 1 is clamped, a first mold that is a mold for primary molding is formed by the lower mold 30 for primary molding and the upper mold 40 for primary molding, and a second mold for secondary molding is formed by the lower mold 60 for secondary molding and the upper mold 70 for secondary molding. Note that an inner diameter (i.e., a diameter of a cavity) of the second mold is larger than an inner diameter (i.e., a diameter of a cavity) of the first mold.

Fig. 2 is a virtual perspective view illustrating a path through which molten resin flows. (a) of Fig. 2 is a virtual perspective view illustrating a path through which molten resin flows in the mold in accordance with Embodiment 1, and (b) of Fig. 2 is a virtual perspective view illustrating a path through which molten resin flows in a mold in accordance with a modification example.

As illustrated in (a) of Fig. 2, the molten resin is introduced to a cavity 51 of the first mold and to a cavity 81 of the second mold via the sprue 22, the runner 12, and a gate 13. Each of the cavity 51 of the first mold and the cavity 81 of the second mold has a substantially cylindrical shape.

The resin molding mold 1 of Embodiment 1 has a configuration in which the runner 12 branches so that the molten resin is introduced to the cavity 51 of the first mold and to the cavity 81 of the second mold via the one (1) runner 12, Note, however, that Embodiment 1 is not limited to this and can employ a configuration in which the first mold and the second mold are separated from each other. That is, as illustrated in (b) of Fig. 2, it is possible to employ a configuration in which (i) molten resin is introduced to the cavity 51 of the first mold via a sprue 22a and a runner 12a which are provided for the first mold and (ii) molten resin is introduced to the cavity 81 of the second mold via a sprue 22b and a runner 12b which are provided for the second mold. Alternatively, the first mold can be a composite mold in which the runner 12a illustrated in (b) of Fig. 2 is caused to branch so that molten resin is introduced to a plurality of cavities 51 in the first mold. Similarly, the second mold can be a composite mold.

The resin molding mold 1 of Embodiment 1 includes the first mold and the second mold. Note, however, that Embodiment 1 is not limited to this and it is possible to employ a resin molding mold including only a first mold and a resin molding mold including only a second mold.

In a case where a tube having a hollow section is formed by resin molding, the resin molding is carried out in a state where a core pin 4 serving as a slide core is inserted in the cavity of the mold as illustrated in (a) of Fig. 1. The core pin 4 is inserted in the cavity so that a central axis of the core pin 4 conforms to a central axis of the cavity.

### <Tube>

Fig. 3 is a view schematically illustrating a tube 90 in accordance with Embodiment 1. (a) of Fig. 3 is a perspective view, (b) of Fig. 3 is a partial lateral view, and (c) of Fig. 3 is a cross-sectional view taken along the line a-a in (b) of Fig. 3. The tube 90 has a wall section 91 and a hollow section 92, and a reinforcing member 93 is embedded in the wall section 91.

Fig. 3 exemplifies the tube 90 of Embodiment 1 in which the reinforcing member 93 that is a spiral-shaped member made up of a plurality of turns of spiral sections (i.e., a coil, a plate-like spring) is embedded in the wall section 91. Note, however, that Embodiment 1 is not limited to this. The tube 90 of Embodiment 1 can be a tube in which a reinforcing member that is (i) a spiral-shaped member made up of at least one turn of a spiral section or (ii) at least one ring-shaped member (which can be a ring or a wide-ring-shaped member) is embedded in a part to be reinforced. Alternatively, the tube 90 can be a tube in which an embedded reinforcing member is a plurality of ring-shaped members which are arranged in an axis direction of the tube 90 at predetermined intervals similar to those of the above a plurality of turns of spiral sections serving as the reinforcing member 93. Alternatively, the reinforcing member can be a member formed by combining a spiral-shaped member and a ring-shaped member.

The tube 90 includes the reinforcing member 93, and therefore the tube 90 is hardly bent in a folded manner and it is consequently possible to prevent the hollow section 92 from becoming narrow even in a case where external force is applied to the tube 90.

### <Mold>

The following description will discuss, with reference to Figs. 4 and 5, the resin molding mold 1 which can be suitably used to produce the tube 90. The resin molding mold 1 of Embodiment 1 includes a first mold 50 and a second mold 80.

Fig. 4 is a view schematically illustrating the first mold 50 in accordance with Embodiment 1. (a) of Fig. 4 is a plan view, (b) of Fig. 4 is a cross-sectional view taken along the line A-A in (a) of Fig. 4, (c) of Fig. 4 is a cross-sectional view taken along the line B-B in (a) of Fig. 4, and (d) of Fig. 4 is an enlarged view illustrating a part C in (b) of Fig. 4. Note that, for convenience of explanation, (c) of Fig. 4 shows configurations of the respective sections in an enlarged manner.

As illustrated in (c) of Fig. 4, the lower mold 30 for primary molding and the upper mold 40 for primary molding which constitute the first mold 50 are cavity type molds. By clamping the lower mold 30 for primary molding and the upper mold 40 for primary molding, the cavity 51 having a substantially cylindrical shape is formed as a hollow part in the first mold 50. Moreover, an inner wall surface 52 of the first mold 50 includes an inner wall surface 31 of the lower mold 30 for primary molding and an inner wall surface 41 of the upper mold 40 for primary molding.

Fig. 5 is a view schematically illustrating the second mold 80 in accordance with Embodiment 1. (a) of Fig. 5 is a plan view, (b) of Fig. 5 is a cross-sectional view taken along the line D-D in (a) of Fig. 5, and (c) of Fig. 5 is a cross-sectional view taken along the line E-E in (a) of Fig. 5.

As illustrated in (c) of Fig. 5, the lower mold 60 for secondary molding and the upper mold 70 for secondary molding which constitute the second mold 80 are cavity type molds. By clamping the lower mold 60 for secondary molding and the upper mold 70 for secondary molding, the cavity 81 having a substantially cylindrical shape is formed as a hollow part in the second mold 80. Moreover, an inner wall surface 82 of the second mold 80 includes an inner wall surface 61 of the lower mold 60 for secondary molding and an inner wall surface 71 of the upper mold 70 for secondary molding.

A diameter of the cavity 81 in the second mold 80 is larger than a diameter of the cavity 51 in the first mold 50. That is, an inner diameter of the second mold 80 is larger than an inner diameter of the first mold 50. Note that the diameter of the cavity 51 of the first mold 50 herein means a diameter of the cavity 51 not including a hole-like recessed part 56 (described later). Note also that the diameter of the cavity 81 of the second mold 80 is equal to or longer than a diameter of the cavity 51 including a hole-like recessed part 56 (described later).

As illustrated in Fig. 4, the inner wall surface 52 of the first mold 50 is provided with at least one hole-like recessed part 56. As illustrated in (b) and (d) of Fig. 4, it is preferable that hole-like recessed parts 56 are discretely (preferably regularly) provided in the inner wall surface 52 of the first mold 50 along one of a plurality of lines parallel to the axis direction of the cavity 51. Further, it is preferable that other plurality of hole-like recessed parts 56 are discretely provided along other one(s) of the plurality of lines. The number of the other one(s) of the plurality of lines is preferably 2 or more, and it is more preferable that the other one(s) of the plurality of lines are arranged equiangularly on the inner wall surface 52, relative to a center of the cavity 51. Furthermore, it is more preferable that a depth of each of the hole-like recessed parts 56 is designed so that a diameter of a virtual cylinder, which is to be circumscribed around the cavity 51 including the hole-like recessed parts 56, is equal to the inner diameter of the second mold 80.

In Embodiment 1, an example is described in which the mold is attached to a vertical injection molding machine. Note, however, that Embodiment 1 is not limited to this and the mold can be attached to a horizontal injection molding machine.

### <Production method>

The following description will discuss a method for producing a tube 90 with use of the resin molding mold 1. The method of Embodiment 1 for producing a tube includes, as an injection molding step, a primary molding step (first step) and a secondary molding step (second step).

In the injection molding carried out in the production method of Embodiment 1, first, molten resin is obtained by heating a moldable material in an injection unit (not illustrated), as with in conventional injection molding. Next, the molten resin is injected into a clamped mold, and then the molten resin is cooled, and thus a molded product is obtained. As a material for injection molding, general thermoplastic resin can be used. Alternatively, it is possible to appropriately select from urethane resin, vinyl chloride resin, polyethylene resin, other thermoplastic elastomer resin, and the like by taking into consideration moldability, a mechanical characteristic demanded of a product, and the like.

In the descriptions below, differences from conventional injection molding will be mainly described in detail.

### (Primary molding step)

In the primary molding step, the reinforcing member 93 and the core pin 4 serving as a slide core are placed in the cavity 51 of the first mold 50. In this case, the reinforcing member 93 is placed so as to cover the core pin 4, and the core pin 4 is arranged so that a central axis of the core pin 4 and a central axis of the cavity 51 become collinear.

Next, molten resin is injected into the cavity 51 of the first mold 50 in a state where the reinforcing member 93 and the core pin 4 are placed in the cavity 51 as above described. In this case, the molten resin flows into the hole-like recessed parts 56 provided on the inner wall surface 52.

Subsequently, the molten resin is cooled and solidified. This results in a primary molded body which includes (i) a first resin layer (tubular resin) which has, on a surface thereof, protrusions corresponding to the hole-like recessed parts 56 and which has a tubular shape and (ii) a reinforcing member 93 which is provided along an outer peripheral surface of the first resin layer. Note that, on a surface of the primary molded body, the reinforcing member 93 can be partially exposed in the outer peripheral surface of the first resin layer having a tubular shape or the reinforcing member 93 does not need to be partially exposed in the outer peripheral surface of the first resin layer.

Note that, although the primary molded body is not illustrated in a drawing, the primary molded body formed by the injection molding has an outer shape that corresponds to a shape of an inner wall of the first mold 50. That is, the outer shape of the primary molded body becomes similar to a shape illustrated in (d) of Fig. 4.

### (Secondary molding step)

In the secondary molding step, the primary molded body and the core pin 4 serving as a slide core are placed in the cavity 81 of the second mold 80. In this case, the primary molded body and the core pin 4 are arranged so that central axes of the primary molded body and the core pin 4 and a central axis of the cavity 81 become collinear. Note that the core pin 4 itself which has been inserted to the cavity 51 in the primary molding step can also be used for the cavity 81 or alternatively another core pin can be used for the cavity 81.

In a case where the primary molded body is placed in the cavity 81, there is a gap between at least part of the surface of the primary molded body and the inner wall surface 82 of the second mold 80 because an inner diameter of the second mold 80 is larger than an inner diameter of the first mold 50.

Next, molten resin is injected into the cavity 81 in a state where the primary molded body and the core pin 4 are placed in the cavity 81 as above described. As already described, there is a gap between at least part of the surface of the primary molded body and the inner wall surface 82. Therefore, by injecting the molten resin into the cavity 81, the primary molded body is to be covered (i.e., surrounded) with the molten resin and the gap is filled with the molten resin.

Subsequently, the molten resin is solidified, and thus a second resin layer is formed outside the first resin layer so as to cover the reinforcing member 93 which has been exposed in the surface of the primary molded body. As such, it is possible to produce a tube 90 in which the reinforcing member 93 is embedded in the wall section 91 (see Fig. 3) by the primary molding step and the secondary molding step.

In a case where the primary molded body is provided in the cavity 81, there is a gap between the primary molded body and the inner wall surface 82 of the second mold 80. This causes the primary molded body to be deformed by its own weight or by injection pressure, and, in particular, causes the primary molded body to be bent at a center part of the second mold 80. This results in the occurrence of decentering in which the central axis of the primary molded body does not match a central axis of the tube which has been completed as a result of the secondary molding step. Consequently, in a longitudinal direction of the second mold 80, a distance between the inner wall surface 82 and the surface of the primary molded body becomes nonuniform. This causes a thickness of a wall section of the tube thus produced to be nonuniform. Furthermore, depending on circumstances, a problem may arise that the reinforcing member is not embedded in the wall section, and therefore ends up being exposed.

However, according to the production method of Embodiment 1, a protrusion is provided on the surface of the primary molded body. Therefore, even in a case where the primary molded body is bent when the primary molded body is placed in the cavity 81 of the second mold 80, the protrusion comes into contact with the inner wall surface 82 of the second mold 80, and therefore reduces bending of the primary molded body.

As compared with an aspect in which no hole-like recessed part 56 is provided in the first mold 50, therefore, it is possible to enhance uniformity of a distance between the inner wall surface 82 of the second mold 80 and the surface of the primary molded body in the longitudinal direction of the second mold 80. This makes it possible to produce a tube 90 in which, in the longitudinal direction, uniformity in thickness of the wall section is improved.

In addition, a depth of each of the hole-like recessed parts 56 is designed so that a diameter of a virtual cylinder, which is to be circumscribed around the cavity 51 including the hole-like recessed parts 56, is equal to the inner diameter of the second mold 80. This allows the diameter of the virtual cylinder, which is circumscribed around the primary molded body, to be equal to the inner diameter of the second mold 80. Therefore, in a case where the primary molded body is placed in the cavity 81 of the second mold 80 so that a protrusion of the primary molded body is in contact with the inner wall surface 82 of the second mold 80, it is possible to restrict, at least in a region around the protrusion, misalignment between the central axis of the primary molded body and the central axis of the cavity 81 of the second mold 80.

Furthermore, in a case where more hole-like recessed parts 56 are discretely provided on the inner wall surface 52 of the first mold 50, a large number of protrusions can be discretely provided on the surface of the primary molded body. This restricts, in a larger area in the cavity 81, misalignment between the central axis of the primary molded body and the central axis of the cavity 81 of the second mold 80. Therefore, it is possible to further enhance uniformity of a distance between the inner wall surface of the second mold 80 and the surface of the primary molded body in the longitudinal direction of the second mold 80. This makes it possible to produce a tube 90 in which, in the longitudinal direction, uniformity in thickness of the wall section is further improved.

As above described, since the protrusion for reducing decentering of the primary molded body in the cavity 81 is provided in the primary molded body, an injection direction of the molten resin in the secondary molding step can be a direction from a base part of the core pin 4 to a tip of the core pin 4 or, conversely, a direction from the tip of the core pin 4 to the base part. This is because of the following reason: that is, in a case where the injection direction from the tip to the base part of the core pin 4 is employed in a mode in which the protrusion is not provided in the primary molded body, decentering of the primary molded body easily occurs, whereas, in a mode in which the protrusion is provided in the primary molded body, the protrusion makes contact with the inner wall surface 82 of the second mold 80 and therefore decentering of the primary molded body hardly occurs.

The above description discussed the method for producing the tube 90 with the use of the first mold 50 in which the hole-like recessed parts 56 are provided on the inner wall surface 52. However, the production method in accordance with Embodiment 1 is not limited to this method, but can be carried out such that a first mold 50, in which no hole-like recessed part 56 is provided on an inner wall surface 52, is used in a case where there is no need to consider restriction of decentering of the primary molded body. Even in such a case, it is not necessary to carry out a step of putting a reinforcing member over a tubular resin unlike the production method of JP 4-174663, and it is therefore possible to produce the tube 90 which includes the reinforcing member 93 in the wall section 91 with the simpler steps. Further, in the production method of Embodiment 1, the injection molding is carried out while the reinforcing member 93 is placed in the cavity 51 of the first mold 50, and therefore no gap is formed between the tubular resin and the reinforcing member 93. From this, an amount of the molten resin that is needed to cover the reinforcing member 93 in the secondary molding step is smaller than that of liquid silicone rubber which is needed in the production method of JP 4-174663. This makes it possible to produce the tube 90 in which the reinforcing member 93 is embedded in the wall section 91, without excessively enlarging an outer diameter of the tube 90 with respect to an inner diameter of the tube 90.

### <Cross section of tube>

In the above described production method, the first resin layer is formed in the primary molding step, and the second resin layer is formed outside the first resin layer in the secondary molding step. Therefore, the wall section 91 of the tube 90 is made up of (i) the first resin layer which is an inner layer and (ii) the second resin layer which is an outer layer.

Moreover, the reinforcing member 93 is provided along a boundary between the first resin layer and the second resin layer. That is, in a cross section (cross-sectional view taken along the line b-b in (c) of Fig. 3) of the tube 90 taken along a plane including an axis direction of the tube 90, cross sections of the reinforcing member 93 are located at positions in the boundary between the first resin layer and the second resin layer.

As illustrated in (d) of Fig. 3, on the surface of the primary molded body obtained in the primary molding step, a protrusion is provided so as to correspond to a hole-like recessed part 56 which is provided on the inner wall surface 52 of the first mold 50. Therefore, in at least part of the cross section of the tube 90, (i) a part of the first resin layer is thicker than other parts of the first resin layer, (ii) a part of the second resin layer is thinner than other parts of the second resin layer, and (iii) a sum of the thickness of the first resin layer in a radial direction and the thickness of the second resin layer in the radial direction is equal to the thickness of the wall section 91.

Note that the first resin layer and the second resin layer can be made of identical resin materials or can be made of respective different resin materials which are compatible with each other.

### [Embodiment 2]

The following description will discuss, with reference to Fig. 6, details of (i) a method for producing a tube in accordance with an embodiment of the present invention and (ii) a mold in accordance with an embodiment of the present invention. For convenience of explanation, identical reference numerals are given to constituent members identical to those described in Embodiment 1, and such constituent members will not be repeatedly described.

### <Mold>

The following description will discuss a resin molding mold 1 which can be suitably used to produce the tube 90 illustrated in Fig. 3. The resin molding mold 1 of Embodiment 2 includes a first mold 150 and a second mold 80 (see Fig. 5).

Fig. 6 is a view schematically illustrating the first mold 150 in accordance with Embodiment 2 of the present invention (a) of Fig. 6 is a plan view, (b) of Fig. 6 is a cross-sectional view taken along the line F-F in (a) of Fig. 6, (c) of Fig. 6 is a cross-sectional view taken along the line G-G in (a) of Fig. 6, and (d) of Fig. 6 is an enlarged view illustrating a part H in (b) of Fig. 6. Note that, for convenience of explanation, (c) of Fig. 6 shows configurations of the respective sections in an enlarged manner.

By clamping a lower mold 130 for primary molding and an upper mold 140 for primary molding, a cavity 151 having a substantially cylindrical shape is formed in the first mold 150 (see (c) of Fig. 6). Moreover, an inner wall surface 152 of the first mold 150 includes an inner wall surface 131 of the lower mold 130 for primary molding and an inner wall surface 141 of the upper mold 140 for primary molding.

As illustrated in Fig. 6, the inner wall surface 152 of the first mold 150 is provided with hole-like recessed parts 156. The hole-like recessed parts 156 each include (i) a first hole-like recessed part 154 and (ii) a second hole-like recessed part 155 which is provided at a part of a bottom surface of the first hole-like recessed part 154. In this way, (i) a hole-like recessed part 156 has surfaces of respective levels in a depth direction, (ii) a bottom surface of a first hole-like recessed part 154 serves as a surface of the shallowest level among the surfaces of the hole-like recessed parts 156, and (iii) a bottom surface of a second hole-like recessed part 155 serves as a bottom surface of the deepest level among the surfaces of the hole-like recessed parts 156. That is, an inner surface of the hole-like recessed parts 156 has a step-like form. This causes an area of the bottom surface of the second hole-like recessed part 155 to be smaller than an opening area of the hole-like recessed parts 156 in a plan view. For example, a size of the second hole-like recessed part 155 in a plan view is preferably small, and is preferably, for example, a size of 1 mm × 1 mm. Alternatively, it is possible to provide a plurality of second hole-like recessed parts 155 on a first hole-like recessed part 154.

### <Production method>

### (Primary molding step)

In a primary molding step, as with the production method of Embodiment 1, a reinforcing member 93 and a core pin 4 (see Fig. 1) are placed in the cavity 151 of the first mold 150. In this case, the reinforcing member 93 is placed so as to cover the core pin 4, and the core pin 4 is arranged so that a central axis of the core pin 4 and a central axis of the cavity 151 become collinear.

Next, molten resin is injected into the cavity 151 of the first mold 50 in a state where the reinforcing member 93 and the core pin 4 are placed in the cavity 151 as above described. In this case, the molten resin flows into the hole-like recessed parts 56 provided on the inner wall surface 152. More specifically, the molten resin flows into the first hole-like recessed parts 154 and into the second hole-like recessed parts 155.

Subsequently, the molten resin is cooled and solidified. This results in a primary molded body which includes (i) a first resin layer (tubular resin) which has, on a surface thereof, protrusions corresponding to the hole-like recessed parts 156 and which has a tubular shape and (ii) a reinforcing member 93 which is provided along an outer peripheral surface of the first resin layer. Each of the hole-like recessed parts 156 includes a first hole-like recessed part 154 and a second hole-like recessed part 155. Therefore, a lower protrusion, which corresponds to the first hole-like recessed part 154, and an upper protrusion, which corresponds to the second hole-like recessed part 155, are formed as a protrusion in the primary molded body. Note that the lower protrusion serves as a bottom part of the protrusion, and the upper protrusion serves as an uppermost part of the protrusion.

### (Secondary molding step)

In a secondary molding step, the primary molded body and the core pin 4 are placed in a cavity of the second mold 80, as with the production method of Embodiment 1. Next, molten resin is injected into the cavity 81 of the second mold 80 in a state where the primary molded body and the core pin 4 are placed in the cavity 81 as above described.

Subsequently, the molten resin is solidified, and thus a second resin layer is formed outside the first resin layer so as to cover the reinforcing member 93 which has been exposed in the surface of the primary molded body. As such, it is possible to produce a tube 90 in which the reinforcing member 93 is embedded in the wall section 91 by the primary molding step and the secondary molding step.

According to the production method of Embodiment 2, as with the production method of Embodiment 1, it is possible to enhance uniformity of a distance between the inner wall surface 82 of the second mold 80 and the surface of the primary molded body in the longitudinal direction of the second mold 80. This makes it possible to produce a tube 90 in which, in the longitudinal direction, uniformity in thickness of the wall section is improved.

In a case where the injection molding of the secondary molding step is carried out in a state where the protrusion is making contact with the inner wall surface 82 of the second mold 80, injected molten resin does not reach an upper surface of the protrusion, and therefore the upper surface of the protrusion is not covered with the molten resin. As a result, an exposed part (corresponding to the protrusion) in which the primary molded body is exposed and a covered part (corresponding to parts other than the protrusion) which is covered with the molten resin in the injection step of the secondary molding step are formed in the surface of the tube 90, and this may impair an appearance of the tube 90.

However, since the first mold 150 includes the second hole-like recessed parts 155, the protrusions on an outer surface of the primary molded body each have (i) a lower protrusion corresponding to the first hole-like recessed part 154 and (ii) an upper protrusion corresponding to a second hole-like recessed part 155. Since the area of the bottom surface of the second hole-like recessed part 155 is smaller than an opening area of a corresponding hole-like recessed part 156 in a plan view, the area of the upper surface of the upper protrusion is smaller than the area of the bottom surface of the protrusion. Therefore, the area of the upper surface of the upper protrusion on the outer surface of the primary molded body is smaller than an area of an upper surface of a protrusion in a case where the protrusion has no upper protrusion. As a result, an area of a part, which makes contact with the inner wall surface 82 of the second mold 80, becomes smaller, as compared with the case where the protrusion has no upper protrusion. From this, a boundary line between an exposed part and a covered part becomes substantially a small circle, and this prevents visual recognition of the boundary line in the surface of the tube 90. As such, in a case where the second hole-like recessed parts 155 are provided in the first mold 250, it is possible to improve the appearance of the tube 90, as compared with a case where no second hole-like recessed part 155 is provided.

Note that the above description discussed the configuration in which the hole-like recessed parts 156 each include a first hole-like recessed part 154 and a second hole-like recessed part 155. However, the configuration of a hole-like recessed part 156 is not limited to this.

The hole-like recessed part 156 does not need to include the second hole-like recessed part 155, provided that an area of its bottom surface is smaller than an area of its opening. For example, the hole-like recessed part 156 can have a shape such as a cone/pyramid shape or a dome shape in which a width decreases toward its bottom. From this, the protrusion provided in the primary molded body has a shape tapering toward the tip. This makes it possible to reduce an area of a part of the primary molded body which part makes contact with the inner wall surface 82 of the second mold 80, and this accordingly prevents visual recognition of the boundary line in the surface of the tube 90. As such, in the aspect in which an area of the bottom surface of the hole-like recessed part 156 is smaller than an opening area of the hole-like recessed part 156, it is possible to improve the appearance of the tube 90, as compared with an aspect in which the area of the bottom surface of the hole-like recessed part 156 is equal to the opening area of the hole-like recessed part 156.

### [Embodiment 3]

The following description will discuss, with reference to Fig. 7, details of (i) a method for producing a tube in accordance with an embodiment of the present invention and (ii) a mold in accordance with an embodiment of the present invention. For convenience of explanation, identical reference numerals are given to constituent members identical to those described in Embodiment 1, and such constituent members will not be repeatedly described.

### <Mold>

The following description will discuss an injection molding mold 1 which can be suitably used to produce the tube 90. The resin molding mold 1 of Embodiment 1 includes a first mold 250 and a second mold 80 (see Fig. 5).

Fig. 7 is a view schematically illustrating the first mold 250 in accordance with Embodiment 3. (a) of Fig. 7 is a plan view, (b) of Fig. 7 is a cross-sectional view taken along the line I-I in (a) of Fig. 7, (c) of Fig. 7 is a cross-sectional view taken along the line J-J in (a) of Fig. 7, and (d) of Fig. 7 is an enlarged view illustrating a part K in (b) of Fig. 7. For explanation, (a) of Fig. 7 illustrates a groove which is provided in an inner wall surface of the first mold 250. Moreover, for explanation, (c) of Fig. 7 illustrates enlarged configurations of the constituent members.

As illustrated in (c) of Fig. 7, by clamping a lower mold 230 for primary molding and an upper mold 240 for primary molding, a cavity 251 having a substantially cylindrical shape is formed in the first mold 250. Moreover, an inner wall surface 252 of the first mold 250 includes an inner wall surface 231 of the lower mold 230 for primary molding and an inner wall surface 241 of the upper mold 240 for primary molding.

In each of the inner wall surface 231 and the inner wall surface 241, a groove corresponding to a shape of the reinforcing member 93 is provided (see (c) of Fig. 7). When clamping is carried out, grooves 253a and 253b which are respectively provided in the inner wall surface 231 and the inner wall surface 241 are combined into a groove 253 which is provided in the inner wall surface 252 of the first mold 250 and corresponds to a spiral shape of the reinforcing member 93.

In the inner wall surface 252 of the first mold 250, a hole-like recessed part 256 is further provided. The hole-like recessed part 256 is provided so as to include at least part of a region located between adjacent portions of the groove 253.

The hole-like recessed part 256 is deeper than the groove 253. Moreover, the hole-like recessed part 256 includes a first hole-like recessed part 254 and a second hole-like recessed part 255 that is provided at a part of a bottom surface of the first hole-like recessed part 254.

The hole-like recessed part 256 can be provided within a space between adjacent portions of the groove 253. Alternatively, the hole-like recessed part 256 can be provided so as to cover (i.e., extend across) at least two adjacent portions of the groove 253.

Here, Fig. 7 shows the example in which the groove has a spiral shape. Note, however, that Embodiment 3 is not limited to this and a shape of the groove can be changed as appropriate in accordance with a shape of the reinforcing member. For example, the groove can have a shape formed by a plurality of ring-shaped grooves or a plurality of wide-ring-shaped grooves which are arranged at regular intervals or at controlled irregular intervals in a direction parallel to a central axis of the cavity. Alternatively, the groove can have a shape formed by combining two or more shapes which are freely selected from a spiral shape, a ring shape, and a wide-ring shape. That is, the hole-like recessed part can be provided so as to include at least part of a region between adjacent sections which are arbitrarily selected from (i) a spiral section corresponding to one turn of a spiral-shaped groove, (ii) a ring-shaped section corresponding to one of ring-shaped grooves, and (iii) a wide-ring-shaped section corresponding to one of wide-ring-shaped grooves.

Here, as illustrated in (d) of Fig. 7, in a case where the hole-like recessed part 256 is provided so as to cover two or more adjacent portions of the groove 253, the groove 253 is interrupted by the hole-like recessed part 256 in the inner wall surface 252. Even in such a case, the hole-like recessed part 256 is assumed to be provided so as to include at least part of a region located between adjacent portions of the groove 253.

The groove 253 has a spiral shape which is formed by a plurality of turns of spiral sections each of which is one turn of the spiral. Therefore, as is clear from the cross-sectional view illustrated in (d) of Fig. 7, the groove 253 includes a plurality of intersections 257 that intersect with a line which is on the inner wall surface 252 and is parallel to a central axis of the cavity 251. Further, the hole-like recessed part 256 is provided so as to at least include a region located between adjacent ones of the plurality of intersections 257.

A size of the second hole-like recessed part 255 in a plan view is preferably small and is preferably, for example, a size of 1 mm x 1 mm. Alternatively, it is possible to provide a plurality of second hole-like recessed parts 255 on a first hole-like recessed part 254. Note that, in Embodiment 3, the second hole-like recessed part 255 is not an essential element in the first mold 250.

As illustrated in (b) and (d) of Fig. 7, a plurality of hole-like recessed parts 256 are discretely (preferably regularly) provided in the inner wall surface 252 of the first mold 250 along one of a plurality of lines parallel to the axis direction of the cavity 251. Further, a plurality of other hole-like recessed parts 256 are discretely provided along other one(s) of the plurality of lines. The number of the other one(s) of the plurality of lines is preferably 2 or more, and it is more preferable that the plurality of lines are arranged equiangularly on a circumference (i.e., on the inner wall surface 252) of the cavity 251, relative to a center of the cavity 251.

### <Production method>

### (Primary molding step)

In a primary molding step, as with the production method of Embodiment 1, a reinforcing member 93 and a core pin 4 (see Fig. 1) are placed in the cavity 251 of the first mold 250. In this case, the reinforcing member 93 is placed so as to be inserted into the groove 253, and the core pin 4 is arranged so that a central axis of the core pin 4 and a central axis of the cavity 51 become collinear.

Next, molten resin is injected into the cavity 251 of the first mold 250 in a state where the reinforcing member 93 and the core pin 4 are placed in the cavity 251 as above described. In this case, the molten resin flows into the hole-like recessed part 256 provided on the inner wall surface 252.

Subsequently, the molten resin is solidified, and thus a primary molded body is obtained which includes (i) a first resin layer (tubular resin) that has a tubular shape and has a protrusion corresponding to the hole-like recessed part 256 and (ii) the reinforcing member 93 that is provided along an outer peripheral surface of the first resin layer.

Further, the hole-like recessed part 256 is provided so as to include a region located between adjacent ones of the intersections 257, and therefore the protrusion is provided so as to include a region located between two intersections at which the reinforcing member 93 having the spiral shape intersects with a line parallel to the central axis of the primary molded body. In other words, the protrusion is provided so as to include a region located between two turns of spiral sections. Moreover, since the hole-like recessed part 256 is deeper than the groove 253, a peak of the protrusion on the surface of the primary molded body is positioned so as to protrude to an outer side than the outer peripheral surface of the reinforcing member 93.

Note that, in a case where the hole-like recessed part 256 of the first mold 250 is provided so as to overlap with (extend across) two or more adjacent portions of the groove 253, it is possible to obtain a primary molded body in which a protrusion is provided so as to cover successive two or more spiral sections. Alternatively, in a case where the hole-like recessed part 256 of the first mold 250 is provided so as to include a region located between adjacent two portions of the groove 253, it is possible to obtain a primary molded body in which a protrusion is provided so as to include a region located between successive two turns of spiral sections.

In an injection step in the primary molding step, the molten resin is injected into the first mold 250 via the gate 13, and is thus poured from one end of the first mold 250 to another end of the first mold 250. In this case, injection pressure is applied to the reinforcing member 93 in a direction from one end of the first mold 250 to another end of the first mold 50. In injection molding using a conventional mold, the reinforcing member may be moved in the cavity due to influence of injection pressure.

On the other hand, in the primary molding step, the reinforcing member 93 is inserted in the groove 253, and it is therefore possible to restrict the movement of the reinforcing member 93, which movement occurs under the influence of injection pressure. This makes it possible to obtain the primary molded body in which the reinforcing member 93 is appropriately provided at an intended position at which the reinforcing member 93 is to be placed on an outer side of the first resin layer.

### (Secondary molding step)

In a secondary molding step, the primary molded body and the core pin 4 are placed in a cavity of the second mold 80, as with the production method of Embodiment 1, Next, molten resin is injected into the cavity 81 of the second mold 80 in a state where the primary molded body and the core pin 4 are placed in the cavity 81 as above described.

Subsequently, the molten resin is solidified, and thus a second resin layer is formed outside the first resin layer so as to cover the reinforcing member 93 which has been exposed in the surface of the primary molded body. As such, it is possible to produce a tube 90 in which the reinforcing member 93 is embedded in the wall section 91 by the primary molding step and the secondary molding step.

In an injection step in the secondary molding step, the molten resin is poured from one end of the second mold 80, at which one end a gate 13 is provided, to another end of the second mold 80. In this case, injection pressure is applied to the reinforcing member 93 in a direction from one end of the second mold 80 to another end of the second mold 80. In injection molding using a conventional mold, the reinforcing member may be moved in the cavity by receiving injection pressure of the molten resin.

On the other hand, according to the secondary molding step, the protrusion is provided so as to include a region located between two turns of spiral sections which are included in the reinforcing member 93. Therefore, movement of the reinforcing member 93 in the axis direction of the primary molded body is restricted by the protrusion, and it is possible to restrict movement of the reinforcing member 93, which movement occurs under the influence of injection pressure.

As such, in the primary molding step, positional displacement of the reinforcing member 93 due to injection pressure is restricted by the groove 253 and further, in the secondary molding step, positional displacement of the reinforcing member 93 due to injection pressure is restricted by the protrusion which is provided so as to include a region located between the spiral sections of the reinforcing member 93: This makes it possible to produce the tube 90 in which a part to be reinforced is reinforced by the reinforcing member 93 that is provided at the intended position with high accuracy, and thus the tube 90 which is hardly bent in a folded manner can be produced.

Further, according to the production method of Embodiment 3, when the primary molded body is placed in the cavity 81 of the second mold 80, the protrusion makes contact with the inner wall surface 82 of the second mold 80, and this reduces decentering of the primary molded body in the cavity 81. From this, in a longitudinal direction of the second mold 80, it is possible to enhance uniformity of a distance between the inner wall surface 82 of the second mold 80 and the surface of the primary molded body, and it is consequently possible to produce the tube 90 in which, in the longitudinal direction, uniformity in thickness of the wall section is improved.

In a case where the injection molding of the secondary molding step is carried out in a state where the protrusion is making contact with the inner wall surface 82 of the second mold 80, injected molten resin does not reach an upper surface of the protrusion, and therefore the upper surface of the protrusion is not covered with the molten resin. As a result, an exposed part (corresponding to the protrusion) in which the primary molded body is exposed and a covered part (corresponding to parts other than the protrusion) which is covered with the molten resin in the injection step of the secondary molding step are formed in the surface of the tube 90, and this may impair an appearance of the tube 90.

However, since the first mold 250 includes the second hole-like recessed parts 255, the protrusions each have (i) a lower protrusion corresponding to the first hole-like recessed part 254 and (ii) an upper protrusion corresponding to a second hole-like recessed part 255. Since the area of the bottom surface of the second hole-like recessed part 255 is smaller than an opening area of a corresponding hole-like recessed part 256 in a plan view, the area of the upper surface of the upper protrusion is smaller than the area of the bottom surface of the protrusion. Therefore, an area of an upper surface of the upper protrusion is smaller than an area of an upper surface of a lower protrusion in a case where the protrusion has no upper protrusion. As a result, an area of a part, which makes contact with the inner wall surface 82 of the second mold 80, becomes smaller, as compared with the case where the protrusion has no upper protrusion. From this, a boundary line between an exposed part and a covered part becomes substantially a small circle, and this prevents visual recognition of the boundary line in the surface of the tube 90. As such, in a case where the second hole-like recessed part 255 is provided in the first mold 250, it is possible to improve the appearance of the tube 90, as compared with a case where no second hole-like recessed part 255 is provided.

### <Cross section of tube>

In the above described production method, the first resin layer is formed in the primary molding step and the second resin layer is formed outside the first resin layer in the secondary molding step. Therefore, as early described with reference to Fig. 3, the wall section 91 of the tube 90 is made up of (i) the first resin layer which is an inner layer and (ii) the second resin layer which is an outer layer.

Moreover, the reinforcing member 93 is provided along a boundary between the first resin layer and the second resin layer. That is, in a cross section of the tube 90 taken along a plane including an axis direction of the tube 90, cross sections of the reinforcing member 93 are located at positions in the boundary between the first resin layer and the second resin layer.

A protrusion (α) which corresponds to the groove 253 in the inner wall surface 252 of the first mold 250 and a protrusion (β) corresponding to the hole-like recessed part 256 are provided on a surface of the primary molded body which has been obtained in the primary molding step, although the first resin layer may not completely cover an outer surface of the reinforcing member 93. As described above, the protrusion (β) is provided so as to include a region located between spiral sections of the reinforcing member 93 having a spiral shape.

Subsequently, the second resin layer is formed around the primary molded body in the secondary molding step, and therefore a recessed part (a) corresponding to the protrusion (a) is formed in the second resin layer which covers the protrusion (a) (see (e) of Fig. 3).

Further, a recessed part (β) corresponding to the protrusion (β) is formed (i) in the second resin layer which covers the protrusion (β) and (ii) so as to include a region located between at least two spiral sections. Specifically, the thickness of the first resin layer (or the second resin layer) varies between (i) a region located between successive two turns of spiral sections and (ii) the other region, and therefore a step is formed between the region and the other region;

From this, in each of the recessed part (a) and the recessed part (β), (i) a part of the first resin layer, which part is in each of the recessed part (a) and the recessed part (β), is thicker than other parts of the first resin layer and (ii) a part of the second resin layer, which part is at each of the recessed part (a) and the recessed part (β), is thinner than other parts of the second resin layer,

The primary molded body in accordance with Embodiment 3 has the protrusions (β) corresponding to the respective hole-like recessed parts 256. Therefore, during an injection molding step for forming the second resin layer around the primary molded body including the first resin layer and the reinforcing member 93, bending (decentering) of the primary molded body can be reduced by causing the protrusions (β) to come into contact with the inner wall surface 82 of the second mold 80 when the primary molded body is placed in the cavity 81 of the second mold 80. This makes it possible to enhance uniformity of a distance between the inner wall surface 82 and the surface of the primary molded body in the longitudinal direction of the second mold 80, and therefore makes it possible to provide a tube 90 in which, in the longitudinal direction, uniformity in thickness of the wall section is improved.

In addition, since the positional displacement of the reinforcing member 93 is restricted by the protrusion (a) corresponding to the groove 253, the tube 90 has a structure in which the reinforcing member 93 is provided at an intended position. This makes it possible to provide a tube 90 in which a part that should be reinforced is reinforced and which is therefore hardly bent in a folded manner.

Moreover, since the positional displacement of the reinforcing member 93 is further restricted by the protrusions (β) corresponding to the respective hole-like recessed parts 256, the tube 90 has a structure in which the reinforcing member 93 is more surely provided at an intended position. As a result, it is possible to provide a tube 90 in which a part that should be reinforced is reinforced with higher accuracy and which is therefore hardly bent in a folded manner.

### [Embodiment 4]

It is possible to employ resin molding that is carried out with a vacuum injection method, instead of the resin molding carried out with the injection molding method which is described in Embodiments 1 through 3. An example configuration of a vacuum injection device which is used to carry out vacuum injection is disclosed in JP 2007 - 13671.

In the vacuum injection method, it is not necessary to inject molten resin into a mold with high pressure, unlike the injection molding method. It is therefore possible to reduce influence on the decentering of a core pin with respect to a central axis of a cavity in the mold.

As resin for use in vacuum injection, it is possible to employ two-part curing silicone resin, two-part curing urethane resin, or the like. A type of hardening reaction can be any of a condensation reaction type and an addition reaction type. It is preferable to employ two-part curing resin of an addition reaction type in which a reduction in weight caused by curing is small (i.e., a type in which a hardening reaction can be facilitated by heating),

### <Mold>

It is possible to employ a mold whose structure is identical with any of those of the resin molding molds 1 used in injection moldings described in Embodiments 1 through 3.

### <Production method>

A tube production method of Embodiment 4 also includes, as a vacuum injection step, a primary molding step (first step) and a secondary molding step (second step), as with the foregoing injection molding step.

In the primary molding step, (i) the mold is placed in a vacuum chamber of the vacuum injection device, (ii) a main agent of two-part curing silicone rubber (resin), which main agent has been prepared in advance, and a curing agent are mixed, (iii) a resultant mixture is poured into a first mold (in which a core pin is being placed) from a sprue having a structure identical with that of the resin molding mold 1, and then (iv) pressure in the vacuum chamber is reduced to a vacuum state. Thus, defoaming is carried out to remove air contained in the resin which has been poured into the first mold. Next, resin that is enough to fill the cavity of the first mold is poured into the first mold, and then the vacuum chamber is opened to atmospheric pressure. This causes the resin to be pressed into and fill the cavity, and a hardening reaction proceeds, and thus a tube (primary molded body) having a predetermined shape can be produced. In order to facilitate the hardening reaction, it is possible to provide a warming device to the mold or the vacuum chamber so as to accelerate curing of the resin which has been poured. In this case, a heating temperature varies depending on a material of the resin and can be, for example, 60°C to 80°C.

In the secondary molding step, the primary molded body and the core pin are placed in a cavity of a second mold having a structure identical with the resin molding mold 1, and vacuum injection is carried out in a manner similar to that described above. Thus, it is possible to produce a tube as an end molded product.

Note that this production method can also be applied to molding carried out with use of a thermosetting elastomer.

### [Main Points]

In order to attain the object, a tube production method in accordance with an aspect of the present invention is a method for producing a tube which includes a wall section, a hollow section, and a reinforcing member, the reinforcing member being provided in the wall section and having a ring shape, a wide-ring shape, or a spiral shape, the method including the steps of: (a) forming a primary molded body, in which the reinforcing member is provided along an outer peripheral surface of a tubular resin, by carrying out resin molding while the reinforcing member and a core pin for forming the hollow section are placed in a cavity of a first mold; and (b) covering the reinforcing member by carrying out resin molding while the primary molded body is placed in a cavity of a second mold which has an inner diameter larger than an inner diameter of the first mold.

Examples of a resin molding method of the present invention encompass an injection molding method and a vacuum injection method.

According to the production method, in the first step (a), the primary molded body can be formed in which the reinforcing member is provided along the outer peripheral surface of the tubular resin. After that, in the second step (b), it is possible to cover the reinforcing member that is provided along the outer peripheral surface of the tubular resin. From this, it is possible to produce the tube which (i) includes the reinforcing member that is provided in the wall section and has a ring shape or a spiral shape and (ii) is hardly bent in a folded manner.

Moreover, it is not necessary to carry out a step of putting a reinforcing member over a tubular resin unlike a conventional production method, and it is therefore possible to produce, with the simpler steps, the tube which includes the reinforcing member that has a ring shape or a spiral shape and is provided in the wall section.

Moreover, in the production method of JP 4-174663, the silicone rubber tube is covered with the reinforcing body, and therefore the reinforcing body is designed to have an inner diameter which is larger than an outer diameter of the silicone rubber tube. As a result, there occurs a problem that an outer diameter of a produced tube becomes excessively larger than an inner diameter of the produced tube.

On the other hand, in the production method of the present invention, the resin molding is carried out while the reinforcing member is placed in the cavity of the first mold, and therefore no gap is formed between the tubular resin and the reinforcing member. As a result, the reinforcing member can be placed with high accuracy with respect to a radial direction of the primary molded body, and it is possible to omit redundant resin wall that is provided on an assumption of an extent to which the reinforcing member may be positionally displaced in the radial direction. From this, it is possible to produce the tube in which the reinforcing member is embedded in a thin wall section.

Note that, in the tube having the above described configuration, any tube-shaped reinforcing member can be suitably used, provided that the reinforcing member is an elastic body whose outline shape is a substantially tubular shape. For example, the reinforcing member can be in any of various forms such as a form of braided product, a form of knitted product, and a form of mesh-like product. Moreover, by adjusting a thickness and intervals of the reinforcing member, it is possible to control easiness in bending the tube and an oblateness of a cross section of the tube.

According to the production method of JP 4-174663, in a case where a core pin covered with a reinforcing body and a silicone rubber tube is provided in a cavity of a mold and then injection molding is carried out, the silicone rubber tube becomes deformed by its own weight or by injection pressure, and, in particular, the silicone rubber tube becomes bent at a center part of the mold. This results in the occurrence of decentering in which a central axis of the silicone rubber tube does not match a central axis of a medical tube which has been completed as a result of the injection molding. Consequently, in a longitudinal direction of the mold, a distance between an inner wall surface of the mold and a surface of the silicone rubber tube becomes nonuniform. This causes a thickness of a wall section of the medical tube thus completed to be nonuniform. Furthermore,
depending on circumstances, a problem may arise that the reinforcing member is not embedded in the wall section, and therefore ends up being exposed.

Therefore, the tube production method in accordance with an aspect 2 of the present invention can be configured so that the first mold has an inner wall surface which is provided with a hole-like recessed part.

According to the production method, a protrusion corresponding to the hole-like recessed part of the first mold is provided on a surface of the primary molded body. Then, by causing the protrusion to come into contact with the inner wall surface of the second mold when the primary molded body is placed in a cavity of the second mold, bending (decentering) of the primary molded body can be reduced. Therefore, as compared with an aspect in which no hole-like recessed part is provided in the first mold, it is possible to enhance uniformity of a distance between the inner wall surface of the second mold and the surface of the primary molded body in a longitudinal direction of the second mold. This makes it possible to produce a tube in which, in the longitudinal direction, uniformity in thickness of the wall section is improved.

Note that in order to further improve the uniformity in thickness of the wall section, it is preferable that, in the inner wall surface of the first mold, at least one hole-like recessed part is provided on each of a plurality of lines parallel to an axis direction of a cavity of the first mold. In addition, it is preferable to discretely provide a plurality of hole-like recessed parts on each of the lines, and it is further preferable to regularly provide the plurality of hole-like recessed parts. It is more preferable that the plurality of lines are arranged equiangularly on a circumference (i.e., on the inner wall surface) of the cavity, relative to a center of the cavity of the first mold.

The tube production method in accordance with an aspect 3 of the present invention can be configured so that a diameter of a virtual cylinder circumscribed around a shape of the cavity of the first mold is equal to the inner diameter of the second mold.

According to the production method, the diameter of a virtual cylinder to be circumscribed around the primary molded body becomes equal to the inner diameter of the second mold. Therefore, in a case where the primary molded body is placed in the cavity of the second mold so that a protrusion of the primary molded body is in contact with the inner wall surface of the second mold, it is possible to restrict, at least in a region around the protrusion, misalignment between the central axis of the primary molded body and the central axis of the cavity of the second mold. This makes it possible to restrict bending of the primary molded body.

Therefore, it is possible to further enhance uniformity of a distance between the inner wall surface of the second mold and the surface of the primary molded body in the longitudinal direction of the second mold. This makes it possible to produce a tube in which, in the longitudinal direction, uniformity in thickness of the wall section is further improved.

In a case where the resin molding is carried out in the second step (b) while the protrusion of the primary molded body is in contact with the inner wall surface of the second mold, resin introduced in the resin introduction step of the second step (b) does not reach an upper surface of the protrusion, and therefore the upper surface of the protrusion is not covered with the resin. As a result, an exposed part (corresponding to the protrusion) in which the primary molded body is exposed and a covered part (corresponding to parts other than the protrusion) which is covered with the resin in the injection step of the second step (b) are formed in the surface of the tube. Therefore, a boundary line between the exposed part and the covered part is visually recognized in the surface of the tube. This is not preferable in terms of appearance.

Therefore, the tube production method in accordance with an aspect 4 of the present invention can be configured so that: the hole-like recessed part has surfaces of respective levels in a depth direction, and includes a first hole-like recessed part and a second hole-like recessed part; a bottom surface of the first hole-like recessed part serves as a surface of a shallowest level among the surfaces of the hole-like recessed part, and a bottom surface of the second hole-like recessed part serves as a bottom surface of a deepest level among the surfaces of the hole-like recessed part, so that an inner surface of the hole-like recessed part has a step-like form; and an area of the bottom surface of the second hole-like recessed part is smaller than an opening area of the hole-like recessed part in a plan view.

According to the production method, the surface of the primary molded body is provided with a protrusion which has (i) a protrusion located at a relatively lower level corresponding to the first hole-like recessed part (hereinafter, such a protrusion will be referred to as "lower protrusion") and (ii) a protrusion located at a relatively higher level corresponding to the second hole-like recessed part (hereinafter, such a protrusion will be referred to as "upper protrusion").

A bottom surface of the first hole-like recessed part serves as a surface of the shallowest level of the hole-like recessed part, and a bottom surface of the second hole-like recessed part serves as a bottom surface of the deepest level of the hole-like recessed part. This (i) causes the lower protrusion to serve as a bottommost part of the protrusion and (ii) causes the upper protrusion to serve as an uppermost part of the protrusion.

Since the area of the bottom surface of the second hole-like recessed part is smaller than the opening area of the hole-like recessed part in a plan view, the area of the upper surface of the upper protrusion is smaller than the area of the bottom surface of the protrusion. That is, an area of an upper surface of the upper protrusion is smaller than an area of an upper surface of a protrusion in a case where the protrusion has no upper protrusion. As a result, an area of a part, which makes contact with the inner wall surface of the second mold, becomes smaller, as compared with the case where the protrusion has no upper protrusion. From this, a boundary line between an exposed part and a covered part becomes short in length (e.g. becomes substantially a small circle), and this prevents visual recognition of the boundary line in the surface of the tube. As such, in a case where the second hole-like recessed part is provided in the mold, it is possible to improve the appearance of a tube which has been completed, as compared with a case where the second hole-like recessed part is not provided.

Note that the protrusion provided on the surface of the primary molded body is not limited to a protrusion having protrusions of two levels; a lower protrusion and an upper protrusion. Alternatively, the protrusion provided on the surface can have protrusions of three or more levels. In such a case also, it is only necessary that (i) an area of an upper surface of an uppermost part (upper protrusion) of projections of multiple levels is smaller than an area of a bottom surface of a projection (lower protrusion) at a bottom part or (ii) the uppermost part of the projections of multiple levels is smaller than the projection at the bottom part.

In order to configure the protrusions of multiple levels, the hole-like recessed part can further include a third hole-like recessed part in addition to the first hole-like recessed part and the second hole-like recessed part. In such a case, it is possible to provide the third hole-like recessed part at a part of the bottom surface of the first hole-like recessed part and to provide the second hole-like recessed part at a part of a bottom surface of the third hole-like recessed part.

In order to attain the object, a mold in accordance with an aspect of the present invention is a mold for forming a tube which includes a wall section, a hollow section, and a reinforcing member, the reinforcing member being provided in the wall section and having a ring shape, a wide-ring shape, or a spiral shape, said mold including: a first mold; and a second mold, an inner diameter of the second mold being larger than an inner diameter of the first mold, and the first mold having an inner wall surface which is provided with a plurality of hole-like recessed parts.

According to the configuration, by carrying out resin molding with the use of the first mold, a primary molded body is obtained so that a protrusion corresponding to the hole-like recessed part of the first mold is provided on the surface of the primary molded body. Then, by causing the protrusion to come into contact with the inner wall surface of the second mold when the primary molded body is placed in a cavity of the second mold, bending (decentering) of the primary molded body can be reduced. Therefore, as compared with an aspect in which no hole-like recessed part is provided in the first mold, it is possible to enhance uniformity of a distance between the inner wall surface of the second mold and the surface of the primary molded body in a longitudinal direction of the second mold. This makes it possible to produce a tube in which, in the longitudinal direction, uniformity in thickness of the wall section is improved.

In order to attain the object, a tube in accordance with an aspect of the present invention is a tube including: a wall section; a hollow section; a reinforcing member which is provided in the wall section and serves as at least one ring-shaped member, at least one wide-ring-shaped member, or at least one turn of a spiral-shaped member; a first resin layer; and a second resin layer which is provided on an outer side of the first resin layer, in a cross section of the tube taken along a plane including an axis direction of the tube, a cross section of the reinforcing member being located in a boundary between the first resin layer and the second resin layer, a part of the first resin layer, which part is located at least at a certain part of the tube, being thicker than other parts of the first resin layer, and a part of the second resin layer, which part is located at least at a certain part of the tube, being thinner than other parts of the second resin layer.

According to the configuration, the protrusion is configured so that, in the cross section of the tube taken along a plane including the axis direction of the tube, a part of the first resin layer, which part is located at least at a certain part of the tube, is thicker than other parts of the first resin layer. Therefore, during a resin molding step with the use of the mold for forming the second resin layer around the primary molded body including the first resin layer and the reinforcing member, bending (decentering) of the primary molded body can be reduced by causing the protrusion to come into contact with the inner wall surface of the mold when the primary molded body is placed in the cavity of the mold. This makes it possible to enhance uniformity of a distance between the inner wall surface of the mold and the surface of the primary molded body in a longitudinal direction of the mold, and therefore makes it possible to provide a tube in which, in the longitudinal direction, uniformity in thickness of the wall section is improved.

The present invention is not limited to the embodiments, but can be altered by a skilled person in the art within the scope of the claims. An embodiment derived from a proper combination of technical means each disclosed in a different embodiment is also encompassed in the technical scope of the present invention.

### Industrial Applicability

The present invention can be used for a purpose of restricting narrowing of a hollow section of a tube, which narrowing occurs as a result of bending. For example, the present invention is applicable to a medical tube.

### Reference Signs List

- 4: Core pin
- 30: Lower mold for primary molding
- 50, 150, 250: First mold
- 51, 151, 251: Cavity
- 52, 152, 252: Inner wall surface
- 80: Second mold
- 81: Cavity
- 90: Tube
- 91: Wall section
- 92: Hollow section
- 93: Reinforcing member
- 56, 156, 256: Hole-like recessed part
- 154, 254: First hole-like recessed part
- 155, 255: Second hole-like recessed part

## Claims

1. A method for producing a tube which includes a wall section, a hollow section, and a reinforcing member, the reinforcing member being provided in the wall section and having a ring shape, a wide-ring shape, or a spiral shape, said method comprising the steps of:
(a)forming a primary molded body, in which the reinforcing member is provided along an outer peripheral surface of a tubular resin, by carrying out resin molding while the reinforcing member and a core pin for forming the hollow section are placed in a cavity of a first mold; and
(b)covering the reinforcing member by carrying out resin molding while the primary molded body is placed in a cavity of a second mold which has an inner diameter larger than an inner diameter of the first mold;
wherein the first mold has an inner wall surface which is provided with a hole-like recessed part.

2. The method as set forth in claim 1, wherein a diameter of a virtual cylinder circumscribed around a shape of the cavity of the first mold is equal to the inner diameter of the second mold.

3. The method as set forth in claim 1 or 2 wherein the resin molding is injection molding.

4. The method as set forth in claim 1 or 2 wherein the resin molding is vacuum injection.

5. The method as set forth in claim 1 or 2, wherein:
- the hole-like recessed part has surfaces of respective levels in a depth direction, and includes a first hole-like recessed part and a second hole-like recessed part;
- a bottom surface of the first hole-like recessed part serves as a surface of a shallowest level among the surfaces of the hole-like recessed part, and a bottom surface of the second hole-like recessed part serves as a bottom surface of a deepest level among the surfaces of the hole-like recessed part, so that an inner surface of the hole-like recessed part has a step-like form; and
- an area of the bottom surface of the second hole-like recessed part is smaller than an opening area of the hole-like recessed part in a plan view.

6. A mold for forming a tube which includes a wall section, a hollow section, and a reinforcing member, the reinforcing member being provided in the wall section and having a ring shape, a wide-ring shape, or a spiral shape, said mold comprising:
- a first mold; and
- a second mold,
- an inner diameter of the second mold being larger than an inner diameter of the first mold, and
- the first mold having an inner wall surface which is provided with a plurality of hole-like recessed parts.

## Patentansprüche

1. Verfahren zum Erzeugen eines Rohrs, das einen Wandabschnitt, einen hohlen Abschnitt und ein Verstärkungselement aufweist, wobei das Verstärkungselement in dem Wandabschnitt vorgesehen ist und eine Ringform, eine breite Ringform oder eine Spiralform hat, wobei das Verfahren die folgenden Schritte umfasst:
(a) Bilden eines geformten Hauptkörpers, in dem das Verstärkungselement entlang einer äußeren umfänglichen Oberfläche eines röhrenförmigen Harzes vorgesehen ist, indem Harzformen ausgeführt wird, während das Verstärkungselement und ein Kernstift zum Bilden des hohlen Abschnitts in einem Hohlraum einer ersten Form platziert sind, und
(b) Abdecken des Verstärkungselements durch Ausführen des Harzformens, während der geformte Hauptkörper in einem Hohlraum einer zweiten Form, die einen Innendurchmesser hat, der größer ist als ein Innendurchmesser der ersten Form, platziert ist;
wobei die erste Form eine Innenwandoberfläche hat, die mit einem lochähnlichen vertieften Teil versehen ist.

2. Verfahren nach Anspruch 1, wobei ein Durchmesser eines virtuellen Zylinders, der um eine Form des Hohlraums der ersten Form umschrieben ist, gleich dem Innendurchmesser der zweiten Form ist.

3. Verfahren nach Anspruch 1 oder 2, wobei das Harzformen ein Spritzgussformen ist.

4. Verfahren nach Anspruch 1 oder 2, wobei das Harzformen ein Vakuumspritzguss ist.

5. Verfahren nach Anspruch 1 oder 2, wobei:
- der lochähnliche vertiefte Teil Oberflächen jeweiliger Niveaus in eine Tiefenrichtung hat und einen ersten vertieften lochähnlichen Teil und einen zweiten vertieften lochähnlichen Teil aufweist;
- eine Bodenoberfläche des ersten lochähnlichen vertieften Teils als eine Oberfläche eines seichtesten Niveaus unter den Oberflächen des lochähnlichen vertieften Teil dient, und eine Bodenoberfläche des zweiten lochähnlichen vertieften Teils als eine Bodenoberfläche eines tiefsten Niveaus unter den Oberflächen des lochähnlichen vertieften Teils dient, so dass eine innere Oberfläche des lochähnlichen vertieften Teils eine stufenähnliche Form hat, und
- eine Fläche der Bodenoberfläche des zweiten lochähnlichen vertieften Teils kleiner ist als eine Öffnungsfläche des lochähnlichen vertieften Teils in einer Draufsicht.

6. Form zum Formen eines Rohrs, das einen Wandabschnitt, einen hohlen Abschnitt und ein Verstärkungselement aufweist, wobei das Verstärkungselement in dem Wandabschnitt vorgesehen ist und eine Ringform, eine breite Ringform oder eine Spiralform hat, wobei die Form Folgendes umfasst:
- eine erste Form, und
- eine zweite Form,
- einen Innendurchmesser der zweiten Form, der größer ist als ein Innendurchmesser der ersten Form, und
- wobei die erste Form eine Innenwandoberfläche hat, die mit einer Vielzahl lochähnlicher vertiefter Teile versehen ist.

## Revendications

1. Procédé de production d'un tube qui inclut une section de paroi, une section creuse et un élément de renforcement, l'élément de renforcement étant prévu dans la section de paroi et ayant une forme annulaire, une forme annulaire large ou une forme spiralée, ledit procédé comprenant les étapes consistant à :
(a) former un corps moulé primaire, dans lequel l'élément de renforcement est prévu le long d'une surface périphérique extérieure d'une résine tubulaire, en effectuant un moulage de résine alors que l'élément de renforcement et une broche de noyau pour former la section creuse sont placés dans une cavité d'un premier moule ; et
(b) couvrir l'élément de renforcement en effectuant un moulage de résine alors que le corps moulé primaire est placé dans une cavité d'un second moule qui a un diamètre intérieur plus grand qu'un diamètre intérieur du premier moule ;
dans lequel le premier moule a une surface de paroi intérieure qui est pourvue d'une partie évidée de type trou.

2. Procédé selon la revendication 1, dans lequel un diamètre d'un cylindre virtuel circonscrit autour d'une forme de la cavité du premier moule est égal au diamètre intérieur du second moule.

3. Procédé selon la revendication 1 ou 2, dans lequel le moulage de résine est un moulage par injection.

4. Procédé selon la revendication 1 ou 2, dans lequel le moulage de résine est une injection sous vide.

5. Procédé selon la revendication 1 ou 2, dans lequel :
- la partie évidée de type trou a des surfaces de niveau respectif dans une direction de profondeur et inclut une première partie évidée de type trou et une seconde partie évidée de type trou ;
- une surface de fond de la première partie évidée de type trou sert de surface d'un niveau le moins profond parmi les surfaces de la partie évidée de type trou et une surface de fond de la seconde partie évidée de type trou sert de surface de fond d'un niveau le plus profond parmi les surfaces de la partie évidée du type trous de sorte qu'une surface intérieure de la partie évidée de type trou ait une forme de type gradin ; et
- une zone de la surface de fond de la seconde partie évidée du type trou est plus petite qu'une zone d'ouverture de la partie évidée de type trou dans une vue en plan.

6. Moule pour former un tube qui inclut une section de paroi, une section creuse et un élément de renforcement, l'élément de renforcement étant prévu dans la section de paroi et ayant une forme annulaire, une forme annulaire large ou une forme spiralée, ledit moule comprenant :
un premier moule ; et
un second moule,
- un diamètre intérieur du second moule étant plus grand qu'un diamètre intérieur du premier moule et
- le premier moule ayant une surface de paroi intérieure qui est pourvue d'une pluralité de parties évidées de type trou.
